# EUROPÄISCHE PATENTSCHRIFT

(11) **EP 1 987 819 B1**
(45) Veröffentlichungstag und Bekanntmachung des Hinweises auf die Patenterteilung: **11.04.2012**
(21) Anmeldenummer: 08010996.0
(22) Anmeldetag: 16.06.2004
(51) Int. Cl.: C07D 213/75

(54) **Injizierbare Darreichungsform von Flupirtin**
Injectable presentation type of flupirtin
Forme enrichie injectable de Flupirtine

(30) Priorität: 20.06.2003 DE 10327674
(43) Veröffentlichungstag der Anmeldung: 05.11.2008
(62) Teilanmeldung aus: 04739918.3
(73) Patentinhaber: AWD.pharma GmbH & Co.KG, 01445 Radebeul (DE)
(72) Erfinder: Pieroth, Michael, 01689 Weinböhla (DE); Stang, Norbert, 01737 Oberhermsdorf (DE); Thoma, Rudy, 14552 Michendorf (DE); Blume, Henning, 61325 Bad Homburg v.d. Höhe (DE)
(74) Vertreter: Eder, Michael

(56) Entgegenhaltungen:
- WO-A-02/080912
- DE-A- 3 416 609

## Beschreibung

Die vorliegende Erfindung betrifft Säureadditionssalze von Flupirtin.

Flupirtin (Katadolon^{®}; 2-Amino-3-carbethoxyamino-6-(4-fluorbenzylamino)-pyridin = 2-Amino-3-ethoxycarbonylamino-6-(p-fluorbenzylamino)-pyridin) ist ein zentral wirksames, nicht-opiates Analgetikum. Flupirtin, insbesondere in Form seines Maleinsäure-Salzes, wird seit vielen Jahren erfolgreich zur Therapie von beispielsweise Nervenschmerzen, Schmerzen bei abnutzungsbedingten Gelenkerkrankungen, Kopfschmerzen und postoperativen Schmerzen eingesetzt. Gemäß DE 41 22 166 A1 kann Flupirtin auch als Mittel zur Bekämpfung von Erkrankungen oder Krankheitssymptomen, die auf Muskelverspannungen beruhen oder eine Folge solcher Muskelverspannungen sind, eingesetzt werden. In der DE 43 27 516 ist des weiteren die Verwendung von Flupirtin zur Behandlung von cerebraler Ischämie und neurodegenerativen Erkrankungen beschrieben. Aus der DE 195 41 405 A1 ist die Verwendung von Flupirtin zur Prophylaxe und Therapie von Erkrankungen, die mit einer Beeinträchtigung des hämatopoetischen Zellsystems einhergehen, bekannt. Die DE 100 48 969 A1 beschreibt ferner die Verwendung von Flupirtin zur Tinnitus-Behandlung. Die Herstellung von Flupirtin und physiologisch verwendbarer Salze davon ist in DE 17 95 858 C2, DE 31 33 519 C2 und DE 34 16 609 A1 beschrieben.

Flupirtin wird hauptsächlich oral appliziert. So beschreibt die DE 93 21 574 U1 beispielsweise Arzneimittel-Formulierungen in Form von Tabletten, Granulaten oder Pellets, die Flupirtinmaleat als Wirkstoff enthalten. Aus der DE 43 19 649 A1 sind feste Flupirtin-haltige orale Darreichungsformen mit kontrollierter Wirkstoffabgabe bekannt.

Aufgrund der guten schmerzstillenden Wirkung von Flupirtin ist es jedoch erstrebenswert, Flupirtin parenteral zu verabreichen, um eine schnelle lokale oder systemische Wirkung zu erzielen. Dem steht jedoch entgegen, dass Flupirtin beziehungsweise physiologisch wirksame Salze davon in wässrigen Lösungen und in den meisten physiologisch verträglichen organischen Lösungsmitteln kaum löslich sind.

Die DE 34 16 609 A1 beschreibt pharmazeutische Formulierungen in Form injizierbarer Flupirtin-Gluconat-Lösungen, die unter Verwendung geeigneter Lösungsmittel hergestellt werden. Als Lösungsmittel wird insbesondere ein Gemisch aus Polyethylenglykol und Wasser oder ein Gemisch aus Glycofurol und Wasser eingesetzt. Die beschriebenen Injektionslösungen weisen jedoch eine Reihe gravierender Nachteile auf. So sind die unter Verwendung der Polyethylenglykol/Wasser- oder Glycofurol/Wasser-Gemische hergestellten Flupirtin-Gluconat-Lösungen extrem hypertonisch und daher nur zur intramuskulären Anwendung geeignet. Bedingt durch den relativ niedrigen pH-Wert von 3,2 bis 3,6 und die eingesetzten Hilfsstoffe wie Natriumdisulfit und Propylenglykol kommt es auch häufig zu Irritationen an der Applikationsstelle. Darüber hinaus weisen die beschriebenen Flupirtin-Gluconat-Lösungen eine nicht, ausreichende physikalische Stabilität auf, da sie nur über einen sehr begrenzten Zeitraum stabil sind und bereits nach wenigen Wochen ein Präzipitationsprozess einsetzt, der die Haltbarkeit des Fertigpräparates deutlich limitiert. Darüber hinaus hat sich herausgestellt, dass die physikalische Stabilität der Flupirtin-Lösungen in hohem Maße auch von der Lagertemperatur abhängig ist. Da die Präzipitation bei tieferen Temperaturen deutlich früher einsetzt, ist es erforderlich, bei der Lagerung der Flupirtin-Lösungen eine Mindesttemperatur von 20°C einzuhalten, um die Lagerungsstabilität zu verbessern. Idealerweise müssten solche Injektionslösungen enthaltenden Ampullen bei Temperaturen von 25°C bis 30°C gelagert werden, was in der Praxis jedoch kaum zu realisieren ist.

Das der vorliegenden Anmeldung zugrunde liegende technische Problem besteht daher darin, Mittel und Verfahren zur Herstellung Flupirtin-haltiger pharmazeutischer Zusammensetzungen bereitzustellen, die zur parenteralen Verabreichung geeignet sind und die nicht die im Stand der Technik bekannten Nachteile parenteraler pharmazeutischer Zusammensetzungen aufweisen, das heißt, insbesondere bei Applikation keine Nebenwirkungen wie Irritationen hervorrufen und darüber hinaus über einen ausreichend langen Zeitraum physikalisch und chemisch stabil sind.

Die vorliegende Erfindung löst das ihr zugrunde liegende technische Problem durch die Bereitstellung des Wirkstoffes Flupirtin als physiologisch verträgliches Salz, das als Lyophilisat zur Herstellung einer parenteral zu verabreichenden pharmazeutischen Zusammensetzung eingesetzt werden kann.

Erfindungsgemäß wurde überraschenderweise gefunden, dass die im Stand der Technik bekannten Nachteile und Probleme bei der Handhabung und Lagerung von Flupirtin-Injektionslösungen durch Verwendung der erfindungsgemäßen Flupirtin-Säureadditionssalze, wenn diese als Lyophilisate vorliegen, zur Herstellung parenteral zu verabreichender, flüssiger Formulierungen vollständig behoben werden können. So führt die Rekonstitution der erfindungsgemäßen Flupirtin-Säureadditionssalze, wenn diese als Lyophilisate vorliegen, in vorteilhafter Weise zu sehr klaren Flupirtin-Lösungen, die über mehrere Stunden hinweg stabil sind und keine Präzipitationsprozesse zeigen, während bei konventionellen Injektionslösungen Flupirtin schon unmittelbar nach Lösungseintritt präzipitiert. Aufgrund ihrer Stabilität sind die unter Verwendung der erfindungsgemäßen Flupirtin-Säureadditionssalze, wenn diese als Lyophilisate vorliegen, hergestellten Flupirtin-Lösungen daher in hervorragender Weise zur parenteraler Verabreichung, insbesondere als Injektions- oder Infusionslösungen einsetzbar.

Ein weiterer überraschender Vorteil der erfindungsgemäßen Flupirtin-Säureadditionssalze, wenn diese als Lyophilisate vorliegen, besteht darin, dass zur Herstellung flüssiger Darreichungsformen rein wässrige Medien eingesetzt werden können. Während bei der Herstellung herkömmlicher Flupirtin-Injektionslösungen keine rein wässrigen Medien verwendet werden können, sondern nur Lösungsmittelsysteme, die einen hohen Anteil an organischen Lösungsmitteln wie Propylenglykol aufweisen, sind die erfindungsgemäßen Flu- pirtin-Säureadditionssalze, wenn diese als Lyophilisate vorliegen, in wässrigen Systemen hervorragend löslich, so dass zum Lösen keine organischen Lösungsmittel oder lösungsvermittelnden Substanzen eingesetzt werden müssen. Die erfindungsgemäßen Flupirtin-Säureadditionssalze, wenn diese als Lyophilisate vorliegen, weisen zudem den Vorteil auf, dass ein Erwärmen beim Lösen des Lyophilisates nicht erforderlich ist, da die erfindungsgemäßen Flupirtin-Säureadditionssalze, wenn diese als Lyophilisate vorliegen, bereits bei Raumtemperatur sehr schnell in Lösung gehen.

Ein weiterer Vorteil besteht darin, dass die erfindungsgemäßen Flupirtin-Säureadditionssalze, wenn diese als Lyophilisate vorliegen, nach Belieben rekonstituiert und/oder verdünnt werden können. So kann das erfindungsgemäße Flupirtin-Säureadditionssalze, wenn diese als Lyophilisate vorliegen, gleichermaßen zur Herstellung von intramuskulären oder intravenösen Injektionslösungen, aber auch zur Zubereitung von Infusionslösungen eingesetzt werden. Dabei kann die rekonstituierte wässrige Zubereitung auch als Zumischung zu üblicherweise verwendeten Infusionslösungen verwendet werden. Diese Form der Anwendung ist vor allem für solche Patienten von Vorteil, für die eine systemische Schmerzbehandlung im Zusammenhang mit anderen therapeutischen Maßnahmen erforderlich ist. Da sich die erfindungsgemäßen Flupirtin-Säureadditionssalze, wenn diese als Lyophilisate vorliegen, in charakteristischer Weise sehr schnell lösen, können die Lyophilisate unmittelbar vor Anwendung rekonstituiert werden.

Im Zusammenhang mit der vorliegenden Erfindung wird unter einem "Lyophilisat" ein Material verstanden, dass durch Trocknen im tiefgefrorenen Zustand im Hochvakuum durch Ausfrieren des Lösungsmittels, das im gefrorenen Zustand verdampft, erhalten wird. Ein auf diese Weise erhaltenes gefriergetrocknete Material ist sehr porös und behält sein ursprüngliches Volumen bei. Stoffwechsel-Funktionen, Enzym-Funktionen und/oder biologische Aktivität des Materials kommen nach Lyophilisierung zum Stillstand.

Im Zusammenhang mit der vorliegenden Erfindung wird unter einer "pharmazeutischen Zusammensetzung" oder einem "Arzneimittel" ein zu diagnostischen, therapeutischen und/oder prophylaktischen Zwecken verwendetes, also ein die Gesundheit eines menschlichen oder tierischen Körpers förderndes oder wiederherstellendes Gemisch verstanden, das mindestens einen natürlichen oder synthetisch hergestellten Wirkstoff umfasst, der die therapeutische Wirkung hervorruft.

Die pharmazeutische Zusammensetzung kann üblicherweise auf dem Fachgebiet verwendete Zusatzstoffe, beispielsweise Stabilisatoren, Fertigungsmittel, Trennmittel, Emulgatoren, Detergentien, Antioxidantien, kuchenbildende Mittel oder andere zur Herstellung pharmazeutischer Zusammensetzungen, insbesondere zur Herstellung flüssiger Darreichungsformen, verwendete Stoffe umfassen.

In bevorzugter Ausführungsform der Erfindung handelt es sich bei der herzustellenden pharmazeutischen Zusammensetzung um eine flüssige pharmazeutische Zusammensetzung zur parenteralen Verabreichung.

Unter einer "parenteral" zu verabreichenden Darreichungsform oder phannazeutischen Zusammensetzung" wird eine sterile pharmazeutische Zusammensetzung verstanden, die unter Umgehung des Magen-Darm-Traktes verabreicht wird. Die Vorteile der parenteralen Verabreichung, insbesondere gegenüber der oralen Verabreichung, bestehen vor allem darin, dass ein sehr schneller Wirkungseintritt möglich ist, dass Nebenwirkungen, wie zum Beispiel Erbrechen oder gastrointestinale Reizungen weitestgehend vermieden werden, dass Wirkstoffe gastrointestinal nicht inaktiviert werden, dass auch Wirkstoffe verabreicht werden können, die im Allgemeinen aus dem Magen-Darm-Kanal ungenügend resorbiert werden, dass der Blutspiegel des verabreichten Wirkstoffes vorausberechenbar ist und dass der sogenannte First-pass-Effekt vermieden wird.

Bei parenteral zu verabreichenden pharmazeutischen Zusammensetzungen handelt es sich insbesondere um Injektions- und Infusionslösungen. "Injektionen" oder "Injektionslösungen" sind Zubereitungen mit kleinen Volumina, insbesondere zwischen 1 und 20 ml, die als Lösung, Suspension oder Emulsion appliziert werden. Bei einer "Infusion" oder "Infusionslösung" werden Volumina, die größer als 100 ml sind, appliziert. Die häufigsten parenteralen Applikationswege sind die intravenöse (i.v.), die intramuskuläre (i.m.) und die subkutane (s.c.) Verabreichung. Die intravenöse Verabreichung ermöglicht die schnelle Zufuhr und Verabreichung von Wirkstoffen, die bei anderen parenteralen Verabreichungswegen gewebereizend wirken. Bei intramuskulären und subkutanen Injektionen muss die Isohydrie und Isotonie beachtet werden, da ansonsten lokale Unverträglichkeitserscheinungen auftreten können.

Erfindungsgemäß ist daher vorgesehen, dass die unter Verwendung der erfindungsgemäßen Flupirtin-Säureadditionssalze, wenn diese als Lyophilisate vorliegen, herzustellende parenteral zu verabreichende pharmazeutische Zusammensetzung eine Injektionslösung oder Infusionslösung ist.

Erfindungsgemäß ist vorgesehen, dass Flupirtin in den Lyophilisaten als physiologisch verträgliches Salz vorliegt, wobei in bevorzugter Ausführungsform der Erfindung das Lyophilisat mindestens 100 mg Flupirtin enthält, wobei sich diese Mengenangabe auf die Flupirtin-Base bezieht.

Unter "physiologisch verträglichen Salzen" von Flupirtin werden insbesondere solche Flupirtin-Salze verstanden, die als Säureadditionssalze vorliegen und die eine therapeutische Breite aufweisen, die durch einen ausreichend großen Abstand der Empfindlichkeitskurven der Flupirtin-Salze für deren therapeutische und letale Wirkung gekennzeichnet ist.

Erfindungsgemäße Säuren zur Herstellung physiologisch verträglicher Flupirtin-Salze, sind ausgewählt aus das Gruppe bestehend aus Sulfonsäuren, sowie Ameisen-, Essig-, Propion-, und Phenylessigsäure. Bevorzugte Sulfonsäuresalze sind Methansulfon-, Ethansulfon-, Hydroxyethansulfon-, Ethylensulfon-, Halogenbenzolsulfon-, Toluolsulfon-, Naphthalinsulfonsäure-Additionssalze.

In bevorzugter Ausführungsform der Erfindung ist das physiologisch verträgliche Flupirtin-Salz daher das Formiat, Acetat, Propionat, Phenylacetat, Methansulfonat, Ethansulfonat, Hydroxyethansulfonat, Ethylensulfonat, Halogenbenzonsulfonat, Toluolsulfonat, oder Naphthalinsulfonat. von Flupirtin.

Erfindungsgemäß ist ferner vorgesehen, dass das Flupirtin-Lyophilisat den Säure-Bestandteil des physiologisch verträglichen Flupirtin-Salzes in einer Menge von 60 mg bis 650 mg, vorzugsweise von 200 mg bis 400 mg, bezogen auf 100 mg Flupirtin, enthält.

In einer bevorzugten Ausführungsform der Erfindung enthält das Flupirtin-Lyophilisat neben dem Flupirtin-Säureadditionssalz zusätzlich mindestens ein kuchenbildendes Mittel. Unter einem "kuchenbildenden Mittel" oder/"Gerüstbildner" wird ein Mittel verstanden, das während und/oder nach Lyophilisierung eines Materials die Bildung eines porösen Kuchens mit sehr großer innerer Oberfläche unterstützt. In bevorzugter Ausführungsform enthält das Flupirtin-Lyophilisat als kuchenbildendes Mittel Mannit, Saccharose oder Glycin. Erfindungsgemäß ist insbesondere vorgesehen, dass das kuchenbildende Mittel in dem Flupirtin-Lyophilisat in einer Menge von 10 mg bis 1000 mg, vorzugsweise von 30 mg bis 300 mg, bezogen auf 100 mg Flupirtin, enthalten ist.

In einer weiteren bevorzugten Ausführungsform der Erfindung ist vorgesehen, dass das Flupirtin-Lyophilisat zusätzlich mindestens ein Antioxidans enthält. Unter "Antioxidantien" werden Hilfsstoffe verstanden, die die Oxidation einer Substanz, insbesondere eines Wirkstoffes hemmen, verzögern oder unterbinden können. Bei Antioxidantien kann es sich um Radikalfänger, leicht oxidierbare Stoffe oder Synergisten handelt. Bei der Oxidation organischer Verbindungen entstehen häufig radikalische Zwischenprodukte. Hilfsstoffe mit sterisch behinderten phenolischen Gruppen können leicht Wasserstoff-Radikale auf diese Zwischenprodukte übertragen, wobei sie selbst stabilere Moleküle bilden. Dadurch wird die oxidative Kettenreaktion unterbrochen. Radikalfänger werden insbesondere in nicht-wässrigen, lypophilen Systemen eingesetzt. In wässrigen Systemen werden hingegen vorrangig leicht oxidierbare Stoffe eingesetzt, wobei die Tatsache ausgenutzt wird, dass jeder zu schützende Stoff ein bestimmtes elektrisches Oxidationspotential besitzt. Das einzusetzendes Antioxidans weist dabei ein deutlich niedrigeres Oxidationspotential als die zu schützende Substanz auf. Bei Zutritt von Sauerstoff wird das Antioxidans dann leichter oxidiert als der zu schützende Stoff. Gleichzeitig wirkt der leicht oxidierbare Hilfsstoff als Protonen-Donator und dadurch stabilisierend. Pharmazeutisch nutzbare Stoffe dieser Art sind vor allem Ascorbinsäure mit einem Normaloxidationspotential von -0,04 V. Hydrogensulfite und Sulfite besitzen ein Normaloxidationspotential von +0,12 V. Bei Synergisten handelt es sich um eine Gruppe von Hilfsstoffen, die die Wirkung von Antioxidanzien entweder durch Regenerierung bereits oxidierter Hilfsstoff-Moleküle, durch Komplexierung von Schwermetallspuren, durch Zersetzung der Peroxide als Zwischenstufen der Oxidation oder durch Einstellen eines oxidationshemmenden pH-Wertes unterstützen.

In bevorzugter Ausführungsform der Erfindung enthält das Lyophilisat als Antioxidans Natriumbisulfit oder Ascorbinsäure. Erfindungsgemäß ist vorgesehen, dass das Antioxidans im Flupirtin-Lyophilisat in einer Menge von 0,5 mg bis 10 mg, besonders bevorzugt in einer Menge von 2 mg bis 5 mg, bezogen auf 100 mg Flupirtin, enthalten ist.

In einer weiteren bevorzugten Ausführungsform der Erfindung ist vorgesehen, dass das Flupirtin-Lyophilisat zusätzlich mindestens ein Detergens enthält. Im Zusammenhang mit der vorliegenden Erfindung wird unter einem "Detergens" eine organische grenzflächenaktive Substanz verstanden, die anionisch, kationisch, ampholytisch oder nichtionisch aufgebaut sein kann. Detergentien werden auch als Tenside bezeichnet. In der Pharmazie werden kationische Tenside hauptsächlich als Konservierungs- oder Desinfektionsmittel eingesetzt. Tenside können auch als W/O- oder O/W-Emulgatoren, Netzmittel, Lösungsvermittler, Schaumstabilisatoren oder Antischaummittel eingesetzt werden. Die Auswahl von Detergentien für bestimmte Aufgaben richtet sich sowohl nach der chemischen Konstitution der hydrophilen und lipophilen Gruppen der als Detergens eingesetzten Verbindung, da diese die Affinitäten zu den vorliegenden Phasen bestimmen, als auch nach den HLB-Werten, aber auch nach den Schmelz- beziehungsweise Erstarrungstemperaturen und nach den Viskositäten.

In bevorzugter Ausführungsform enthält das Lyophilisat als Detergens ein Polyvinylpyrrolidon. Bei den Polyvinylpyrrolidonen handelt es sich um Polymerisationsprodukte des Vinylpyrrolidons. Handelsüblich ist eine Reihe von Fraktionen mit unterschiedlichen Molekül-Größen beziehungsweise Molekül-Kettenlängen. Eine hervorstechende Eigenschaft der Polyvinylpyrrolidone ist die gute Löslichkeit sowohl in Wasser als auch in polaren organischen Lösungsmitteln wie Alkoholen, Glykolen etc.. Erfindungsgemäß ist insbesondere vorgesehen, dass das Detergens, insbesondere Polyvinylpyrrolidon, im Flupirtin-Lyophilisat in einer Menge von 10 mg bis 150 mg, besonders bevorzugt in einer Menge von 10 mg bis 50 mg, bezogen auf 100 mg Flupirtin enthalten ist.

Die vorliegende Erfindung beschreibt ebenfalls die Verwendung des Flupirtin-haltigen Lyophilisates zur Herstellung einer parenteral zu verabreichenden pharmazeutischen Zusammensetzung. Es ist dabei vorgesehen, dass das Lyophilisat zur Herstellung der parenteral zu verabreichenden pharmazeutischen Zusammensetzung verwendet wird, indem das Lyophilisat in einem wässrigen Medium und/oder einem organischen Lösungsmittel gelöst wird, wobei die parenteral zu verabreichende pharmazeutische Zusammensetzung erhalten wird. Es ist insbesondere vorgesehen, dass das Lyophilisat dabei bei Raumtemperatur gelöst wird. Als wässriges Medium wird bevorzugt Wasser, besonders bevorzugt Wasser für Injektionszwecke verwendet. In einer anderen Ausführungsform ist vorgesehen, dass als wässriges Medium eine geeignete Pufferlösung verwendet wird. In einer weiteren Ausführungsform ist vorgesehen, dass das Lyophilisat zur Herstellung der parenteralen pharmazeutischen Zusammensetzung in einem Wasser-Lösungsmittel-Gemisch gelöst wird.

Die vorliegende Erfindung betrifft ebenfalls ein Verfahren zur Herstellung einer parenteral zu verabreichenden Flupirtin-haltigen pharmazeutischen Zusammensetzung, wobei ein Flupirtin-Säureadditionssalz-haltiges Lyophilisat in einem wässrigen Medium und/oder einem organischen Lösungsmittel gelöst und eine gebrauchsfertige flüssige pharmazeutische Zusammensetzung erhalten wird.

Vorzugsweise wird das Flupirtin-Lyophilisat bei Raumtemperatur gelöst. In besonders bevorzugter Ausführungsform wird das Flupirtin-Lyophilisat in Wasser, insbesondere Wasser für Injektionszwecke, gelöst. Das Flupirtin-Lyophilisat kann aber auch in einer Pufferlösung oder in einem Wasser-Lösungsmittel-Gemisch gelöst werden.

Über das zum Auflösen verwendete Volumen des wässrigen Mediums kann die Isotonie der erhaltenen Lösung eingestellt werden. Vor der Applikation der herzustellenden parenteralen pharmazeutischen Zusammensetzung kann entschieden werden, wie das Lyophilisat zu rekonstituieren ist und ob gegebenenfalls eine Verdünnung erfolgen kann. So lässt sich aus dem Lyophilisat gleichermaßen eine intramuskuläre oder intravenöse Injektion herstellen. Die rekonstituierte wässrige Zubereitung kann auch als Zumischung zu gängigen Infusionslösungen verwendet werden.

Es ist daher vorgesehen, dass die unter Verwendung des erfindungsgemäßen Verfahrens hergestellte parenteral zu verabreichende pharmazeutische Zusammensetzung eine Injektionslösung ist. Wenn die herzustellende Injektionslösung intravenös appliziert werden soll, ist vorgesehen, dass das Lyophilisat, das vorzugsweise 100 mg Flupirtin an der Form eines Säureadditionssalzes enthält, in 3 bis 20 ml, vorzugsweise 9 bis 15 ml Wasser für Injektionszwecke, Pufferlösung oder Wasser/Lösungsmittel-Gemisch gelöst wird. Wenn die herzustellende Injektionslösung intramuskulär appliziert werden soll, ist vorgesehen, dass das Lyophilisat, das vorzugsweise 100 mg Flupirtin als Säureadditionssalz enthält, in 3 ml Wasser für Injektionszwecke, Pufferlösung oder Wasser/Lösungsmittel-Gemisch gelöst wird. In einer weiteren Ausführungsform der ist vorgesehen, dass die parenteral zu verabreichende pharmazeutische Zusammensetzung eine Infusionslösung ist.

Die vorliegende Erfindung beschreibt ebenfalls ein Verfahren zur Herstellung des Flupirtin-Säureadditionssalz-haltigen Lyophilisates, umfassend
a) Herstellung einer Flupirtin-Lösung durch Zugabe von Flupirtin-Base zu einer wässrigen Lösung der entsprechenden Säure und Lösen darin, und
b) Gefriertrocknung der erhaltenen Flupirtin-Säureadditionssalz-Lösung.

wobei die Säure ausgewählt ist aus der Gruppe bestehend aus Ameisen-, Essig-, Propion-, Phenylessig-, Methansulfon-, Ethansulfon-, Hydroxyethansulfon-, Ethylensulfon-, Halogenbenzolsulfon-, Toluolsulfon-, und Naphthalinsulfonsäure.

In bevorzugter Ausführungsform der Erfindung wird die zum Lösen der Flupirtin-Base verwendete wässrige SäureLösung, vor Zugabe der Flupirtin-Base auf eine über der Raumtemperatur liegende Temperatur erhitzt und bei dieser Temperatur gehalten. Erst nach Erwärmen wird Flupirtin dem erwärmten wässrigen Medium zugegeben und darin gelöst. In bevorzugter Ausführungsform wird das wässrige Medium auf eine Temperatur von 30°C bis 90°C, besonders bevorzugt 70°C erhitzt. Erfindungsgemäß ist ferner vorgesehen, dass Flupirtin unter Rühren in das vorzugsweise erwärmte wässrige Medium gegeben wird. Die Lösung wird dann solange gerührt, bis Flupirtin vollständig gelöst ist.

Erfindungsgemäß ist ferner vorgesehen, dass die so hergestellte Flupirtin-Lösung anschließend filtriert wird. Besonders bevorzugt wird dabei ein Filter mit einer Porenweite von 0,2 µm eingesetzt. Danach wird die vorzugsweise filtrierte Flupirtin enthaltene Lösung in Gefriertrocknungsflaschen eingefüllt, die dann mit Gefriertrockungsstopfen versehen werden. Zum Einfrieren der Flupirtin-Lösung werden die Gefriertrocknungsflaschen bei -45°C gelagert.

Es ist vorgesehen, dass die eigentliche Gefriertrocknung eine Haupttrocknung und eine Nachtrocknung umfasst. In bevorzugter Ausführungsform erfolgt die Haupttrocknung bei einer Temperatur von -37°C bis -23°C und einem Druck von 10 bis 100 mbar. In bevorzugter Ausführungsform erfolgt danach die Nachtrocknung bei einer Temperatur von 27°C und einem Druck von 0,0001 mbar. Nach der Gefriertrocknung wird der Gefriertrockner mit N₂ entspannt. Die das Flupirtin- Lyophilisat enthaltenden Flaschen werden dann vorzugsweise unter einer Stickstoffatmosphäre steril verschlossen.

Die vorliegende Erfindung beschreibt ebenfalls die flüssige pharmazeutische Zusammensetzung zur parenteralen Verabreichung, die durch die Flupirtin-Lyophilisate erhältlich sind.

Die vorliegende Erfindung wird durch die nachfolgenden Beispiele näher erläutert.

### Beispiel 1: Herstellung eines Flupirtin-haltigen Lyophilisates

7,81 g Gluconsäure-d-lacton werden in 70 ml Wasser gelöst und dann auf 70°C erhitzt. Bei dieser Temperatur werden unter Rühren 3,33 g Flupirtin zugegeben und bis zur vollständigen Lösung gerührt. Das so erhaltene Gemisch wird durch einen Filter mit einer Porenweite von 0,2 µm filtriert. Nach der Filtration wird die Lösung in gefriergetrocknete Flaschen abgefüllt und mit geeigneten Gefriertrocknungsstopfen versehen. Zum Gefrieren des Füllgutes werden die Flaschen bei - 45°C gelagert. Die Haupttrocknung des Gefriertrocknungsprozesses erfolgt bei - 37°C bis -23°C und 100 bis 10 mbar. Die Nachtrocknung wird dann bei 27°C und 0,0001 mbar durchgeführt. Nach der Trocknung wird der Gefriertrockner mit N₂ entspannt. Die Flaschen werden dann unter Stickstoff-Atmosphäre verschlossen.

### Beispiel 2: Herstellung eines Flupirtin-haltigen Lyophilisates

7,81 g Gluconsäure-d-lacton werden in 70 ml Wasser gelöst und auf 70°C erhitzt. Bei dieser Temperatur werden unter Rühren 4,0 g Mannit und 3,33 g Flupirtin zugegeben und solange gerührt, bis die zugegebenen Verbindungen vollständig gelöst sind. Das so erhaltene Gemisch wird durch einen Filter mit einer Porenweite von 0,2 µm filtriert. Nach der Filtration wird die Lösung in Gefriertrocknungsflaschen abgefüllt und mit geeigneten Gefriertrocknungsstopfen versehen. Zum Gefrieren des Füllgutes werden die Flaschen bei -45°C gelagert. Die Haupttrocknung des Gefriertrocknungsprozesses erfolgt bei -37°C bis -23°C und 100 bis 10 mbar. Die Nachtrocknung wird bei 27°C und 0,0001 mbar durchgeführt. Nach der Trocknung wird der Gefriertrockner mit N₂ entspannt. Danach werden die Flaschen unter Stickstoff-Atmosphäre verschlossen.

### Beispiel 3: Herstellung eines Flupirtin-haltigen Lyophilisates

7,81 g Gluconsäure-d-lacton werden in 70 ml Wasser gelöst und auf 70°C erhitzt. Bei dieser Temperatur werden unter Rühren 7,5 g Saccharose, 0,4 g Polyvinylpyrrolidon (MG etwa 11500; Kollidon PF17; BASF) und 3,33 g Flupirtin zugegeben. Die Lösung wird solange gerührt, bis die zugegebenen Substanzen vollständig gelöst sind. Das so erhaltene Gemisch wird durch einen Filter mit einer Porenweite von 0,2 µm filtriert. Nach der Filtration wird die Lösung in Gefriertrocknungsflaschen abgefüllt und mit geeigneten Gefriertrocknungsstopfen versehen. Zum Gefrieren des Füllgutes werden die Flaschen bei -45°C gelagert. Die Haupttrocknung des Gefriertrocknungsprozesses erfolgt bei -37°C bis -23°C und 100 bis 10 mbar. Die Nachtrocknung wird bei 27°C und 0,0001 mbar durchgeführt. Nach der Trocknung wird der Gefriertrockner mit N₂ entspannt. Die Flaschen werden dann unter Stickstoffatmosphäre verschlossen.

### Beispiel 4: Zusammensetzung eines Flupirtin-Lyophilisates

Eine Flasche enthält:
100,00 mg Flupirtin
257,85 mg Gluconsäure
300,00 mg Mannit.

### Beispiel 5: Zusammensetzung eines Flupirtin-Lyophilisates

Eine Flasche enthält:
100,00 mg Flupirtin
257,85 mg Gluconsäure
225,00 mg Saccharose
12,00 mg Polyvinylpyrrolidon (Kollidon 17 PF, BASF).

### Beispiel 6: Zusammensetzung eines Flupirtin-Lyophilisates

Eine Flasche enthält:
100,00 mg Flupirtin
257,85 mg Gluconsäure
120,00 mg Mannit
4,50 mg Natriumbisulfit

### Beispiel 7: Zusammensetzung eines Flupirtin-Lyophilisates

Eine Flasche enthält:
100,00 mg Flupirtin
257,85 mg Gluconsäure
207,00 mg Mannit

### Beispiel 8: Herstellung einer flüssigen pharmazeutischen Zusammensetzung

Eine Flasche des in Beispiel 7 beschriebenen Lyophilisats wird mit 3 ml Wasser für Injektionszwecke versetzt. Nach gelegentlichem Umschwenken wird nach etwa 1 min eine klare Lösung erhalten. Die Lösung ist mit 980 mosmol/kg deutlich hypertonisch.

### Beispiel 9: Herstellung einer flüssigen pharmazeutischen Zusammensetzung

Eine Flasche des Lyophilisats aus Beispiel 7 wird mit 9 ml Wasser für Injektionszwecke versetzt. Unter gelegentlichem Rühren wird nach etwa 1 Minute eine klare Lösung erhalten. Die Lösung ist mit 305 mosmol/kg nahezu isotonisch.

## Patentansprüche

1. Säureadditionssalz von 2-Amino-3-carbethoxyamino-6-p-fluor-benzylamino-pyridin [Flupirtin], **dadurch gekennzeichnet, dass** die addierte Säure unter Ameisensäure, Essigsäure, Propionsäure, Phenylessigsäure sowie Sulfonsäuren gewählt ist.

2. Säureadditionssalz gemäß Anspruch 1, weiter **gekennzeichnet dadurch, dass** die addierte Säure unter Ameisensäure, Essigsäure, Propionsäure, Phenylessigsäure, Methansulfonsäure, Ethansulfonsäure, Hydroxyethansulfonsäure, Ethylensulfonsäure, Halogenbenzolsulfonsäure, Toluolsulfonsäure und Naphthalinsulfonsäure gewählt ist.

3. Säureadditionssalz gemäß Anspruch 1 oder 2, weiter **gekennzeichnet dadurch, dass** die addierte Säure unter Essigsäure, Propionsäure, Methansulfonsäure, Ethansulfonsäure und Toluolsulfonsäure gewählt ist.

## Claims

1. Acid addition salt of 2-amine-3-carbethoxyamine-6-p-fluoro-benzylamine-pyridine [Flupirtine], **characterized in that** the added acid is chosen from formic acid, acetic acid, propionic acid, phenylacetic acid and sulfonic acids.

2. Acid addition salt according to claim 1, further **characterized in that** the added acid is chosen from formic acid, acetic acid, propionic acid, phenylacetic acid, methanesulfonic acid, ethanesulfonic acid, hydroxyethanesulfonic acid, ethylenesulfonic acid, halogen benzenesulfonic acid, toluenesulfonic acid and naphthalenesulfonic acid.

3. Acid addition salt according to claim 1 or 2, further **characterized in that** the added acid is chosen from acetic acid, propionic acid, methanesulfonic acid, ethanesulfonic acid and toluenesulfonic acid.

## Revendications

1. Sel d'addition acide de 2-amino-3-carbéthoxyamino-6-p-fluor-benzylamino-pyridine [flupiritine] **caractérisé par le fait que** l'acide additionné est sélectionné parmi l'acide formique, l'acide acétique, l'acide propionique, l'acide acétique phénylique ainsi que les acides sulfoniques.

2. Sel d'addition acide selon la revendication 1 **caractérisé en outre par le fait que** l'acide additionné est sélectionné parmi l'acide formique, l'acide acétique, l'acide propionique, l'acide acétique phénylique, l'acide méthanesulfonique, l'acide éthansulfonique, l'acide hydroxyéthanesulfonique, l'acide éthylènesulfonique, l'acide benzènesulfonique halogéné, l'acide toluènesulfonique et l'acide naphtalènesulfonique.

3. Sel d'addition acide selon la revendication 1 ou 2 **caractérisé en outre par le fait que** l'acide additionné est sélectionné parmi l'acide acétique, l'acide propionique, l'acide méthane sulfonique, l'acide éthanesulfonique et l'acide toluènesulfonique.
